# FASCICULE DE BREVET EUROPEEN

(11) **EP 2 186 544 B1**
(45) Date de publication et mention de la délivrance du brevet: **10.07.2013**
(21) Numéro de dépôt: 09173433.5
(22) Date de dépôt: 19.10.2009
(51) Int. Cl.: A61Q 13/00, A61Q 15/00, A61K 8/25, A61K 8/26, A61K 8/28

(54) **Utilisation de particules minérales amorphes expansées pour augmenter la rémanence d'un parfum ; composition parfumante, procédé de traitement des odeurs corporelles.**
Verwendung von amorphen expandierten Partikeln zur Verbesserung der Haftung eines Parfüms; Duftstoffzusammensetzung; Verfahren zur Behandlung von Körpergerüchen.
Use of amorphous mineral expanded particles for improving perfume tenacity; perfuming composition thereof and process for treating human body odours using said composition.

(30) Priorité: 17.11.2008 FR 0857790
(43) Date de publication de la demande: 19.05.2010
(73) Titulaire: L'Oréal, 75008 Paris (FR)
(72) Inventeur: Aubrun, Odile, 92160, Antony (FR); Cassier, Matthieu, 75017, Paris (FR)
(74) Mandataire: Miszputen, Laurent

(56) Documents cités:
- EP-A1- 0 770 373
- DE-A1- 3 824 940
- DE-A1- 10 356 692
- DE-B- 1 289 600
- FR-A1- 2 722 101
- GB-A- 1 057 316
- SU-A3- 1 790 601
- DATABASE WPI Week 1986 Thomson Scientific, London, GB; AN 1986-153401 XP002538080 "Mixing granular perfumes to give desired aroma - by allowing different colour, porous particulates to hold perfumes corresp. to colours and mixing granular perfumes" & JP 61 087618 A (HIRAO M.) 6 mai 1986 (1986-05-06)
- DATABASE WPI Week 1980 Thomson Scientific, London, GB; AN 1981-04752D XP002538081 "Deodorant with high active at normal temp. - comprises hydrated glyoxal and expanded vermiculite in powder, paste or slurry form etc." & JP 55 148560 A (CHISSO CORP) 19 novembre 1980 (1980-11-19)
- DATABASE WPI Week 1993 Thomson Scientific, London, GB; AN 1993-410773 XP002538082 "umigant perfume compsn. emitting little smoke on burning - comprises base of e.g. coal ash, active alumina or silica gel supporting the perfume" & JP 05 310546 A (KUJU DO KOHO KK; TAIYO KAGAKU KK) 22 novembre 1993 (1993-11-22)

## Description

L'invention a pour objet un procédé cosmétique de parfumage d'une matière kératinique humaine consistant à appliquer sur ladite matière une composition parfumante comprenant dans un milieu cosmétiquement acceptable au moins 0,3% en poids d'au moins une substance parfumante et au moins des particules d'un matériau minéral expansé amorphe **constitué** et de perlite expansée ayant une taille de particules definie par un diamétre médian D50 allant de 0,5 à 50 µm.

La présente invention a pour objet également un procédé cosmétique de traitement des odeurs corporelles humaines en particulier axillaires, consistant à appliquer sur la surface de la peau une quantité efficace de la dite composition.

L'invention se rapporte à des composition parfumantes particulières comprenant dans un milieu cosmétiquement acceptable :
a) au moins 0,3% en poids par rapport au poids total de la composition d'une substance parfumante ;
b) au moins les dites particules.

On sait qu'un parfum est l'association de différentes substances odorantes qui s'évaporent à des périodes différentes. Chaque parfum présente ce que l'on appelle une « note de tête » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »). La persistance de la note de fond correspond à la rémanence du parfum.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Parmi les critères importants de qualité que l'on recherche dans les produits parfumants, figurent d'une part la rémanence du parfum à savoir la persistance de la note de fond sur la peau et d'autre part la transparence de la formulation ainsi que son caractère fluide pour des raisons esthétiques et de confort d'application.

Il également connu de mettre des parfums dans des compositions déodorantes destinées à traiter les odeurs corporelles humaines notamment axillaires.

Il subsiste le besoin de trouver de nouveaux types d'agents permettant d'augmenter efficacement la rémanence du parfum dans une large gamme de produits parfumants comme les eaux de toilette, les eaux de parfum, les produits déodorants et les produits anti-transpirants sans les inconvénients évoqués ci-dessus et sans nuire au confort du consommateur comme le toucher.

On sait que la perlite non expansée et non broyée de taille moyenne allant de 0,1 à 15 mm a été proposée dans le brevet DE3824940 comme matrice incorporant des essences, des arômes ou des désinfectants. Cependant ce type de matériau est difficilement formulable dans les sprays et aérosols du fait qu'il a tendance à boucher les dispositifs de pulvérisation. Ce type de matériau n'est pas très satisfaisant du point de vue cosmétique sensoriel vis à vis du consommateur en raison de son caractère très abrasif.

La demanderesse a découvert de manière surprenante que cet objectif pouvait être atteint en utilisant des particules d'un matériau minéral amorphe expansé **constitué** de perlite expansée dans une composition parfumante comprenant dans un milieu cosmétiquement acceptable au moins 0,3% en poids par rapport au poids total de la composition d'une substance parfumante.

L'invention a pour objet d'une part un procédé cosmétique de parfumage d'une matière kératinique humaine consistant à appliquer sur ladite matière une composition parfumante comprenant dans un milieu cosmétiquement acceptable au moins 0,3% en poids d'au moins une substance parfumante et au moins des particules d'un matériau minéral expansé amorphe **constitué** de perlite expansées **ayant une taille de particule définie par un diamètre médian D₅₀ allant de 0,5 à 50 µm**.

La présente invention a pour objet également un procédé cosmétique de traitement des odeurs corporelles humaines en particulier axillaires, consistant à appliquer sur la surface de la peau une quantité efficace de ladite composition.

Par « matière kératinique humaine », on entend la peau, les cheveux, le cuir chevelu, les cils, les sourcils, le cuir chevelu, les ongles, les lèvres.

Par « substance parfumante », on entend tout parfum ou arôme susceptible de dégager une odeur agréable.

On entend par « milieu cosmétiquement acceptable » dans la composition de l'invention, un milieu non toxique et susceptible d'être appliqué sur la peau (y compris l'intérieur des paupières), les lèvres, les ongles ou les cheveux, les cils et les sourcils d'êtres humains.

Par « matériau minéral », on entend au sens de l'invention, tout matériau constitué de substances inorganiques.

Par « amorphe », on entend par tout matériau comportant moins de 10% en poids de phase cristalline et de préférence moins de 5% en poids de phase cristalline voire totalement non cristallisé et ne possédant pas de structure atomique ordonnée.

Par «matériau expansé », on entend tout matériau ayant une densité apparente non tassée à 25°C allant de 10 à 400 kg/m³ (Norme DIN 53468). Cette densité peut notamment être le résultat d'un traitement par un procédé thermique notamment à une température allant de 700 à 1500°C et de préférence de 800 à 1100°C.

Comme substance parfumante, on peut utiliser dans la composition de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance parfumante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcool aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On utilise de préférence comme substance parfumante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Selon un mode préféré de réalisation de l'invention, on utilise un mélange de différentes substances parfumante qui engendrent en commun une note plaisante pour l'utilisateur. Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges

De préférence, la quantité de substance(s) parfumante(s) varie de 0,5 à 30 % en poids, mieux de 1 à 25% en poids par rapport au poids total de la composition

### MATERIAU MINERAL AMORPHE EXPANSE

Le matériau minéral amorphe expansé constitué de perlite conforme à l'invention contient au moins deux éléments choisis parmi le silicium, l'aluminium et le magnésium.

Le matériau minéral expansé issu d'au moins une roche volcanique conforme à la présente invention contient généralement dans sa composition au moins deux éléments choisis parmi le silicium, l'aluminium et le magnésium, Il est susceptible d'être obtenu par expansion thermique d'une roche volcanique comprenant de 1 à 10% en poids d'eau et de préférence 1 à 5% en poids d'eau et moins de 10% en poids de roche cristalline par rapport au poids total de la composition de la roche suivie de préférence par un broyage. La température du procédé d'expansion peut varier de 700 à 1500°C et de préférence de 800 à 1100 °C. On peut notamment utiliser le procédé d'expansion décrit dans le brevet US 5,002,698.

Les roches volcaniques ou « effusives » sont généralement produites par le refroidissement rapide du liquide magmatique au contact de l'air ou de l'eau (phénomène de trempe donnant une roche hyaline). Les roches volcaniques utilisables selon la présente invention sont choisies parmi celles définies selon la classification de Streckeisen (1974).

Selon un mode particulier de l'invention, les particules de matériau minéral amorphe expansé présentent un coefficient d'expansion de 2 à 70.

De manière préférentielle, les particules de matériau amorphe minéral amorphe expansé présentent une densité apparente non tassée à 25°C allant de 10 à 300 kg/m³ (Norme DIN 53468).

Selon un mode particulier de l'invention, les particules de matériau minéral amorphe expansé présentent un pH spontané mesuré à 25°C dans une dispersion dans l'eau à 10% en poids allant de 6 à 8.

Selon un autre mode particulier de l'invention, les particules de matériau minéral amorphe expansé présentent un teneur en silice supérieure ou égale à 65% en poids par rapport au poids total de la composition du matériau.

Selon un autre mode particulier de l'invention, les particules de matériau minéral amorphe expansé présentent une taille de particules définie par un diamètre médian D₅₀ allant de 0,5 à 50 µm.

De manière préférentielle, les particules de matériau minéral amorphe expansé présentent une forme plaquettaire.

Selon l'invention, les particules de matériau amorphe expansé **sont** des particules de perlite expansée.

Les perlites utilisables selon l'invention sont généralement des aluminosilicates d'origine volcanique et ont comme composition
70,0-75,0% en poids de silice SiO₂
12,0-15,0% en poids d'oxyde d'aluminium oxyde Al₂O₃
3,0-5,0% d'oxyde de sodium Na₂O
3,0-5,0% d'oxyde de potassium K₂O
0,5-2% d'oxyde de fer Fe₂O₃ →
0,2-0,7% d'oxyde de magnésium MgO
0,5-1,5% d'oxyde de calcium CaO
0,05 - 0,15% d'oxyde de titane TO₂

La perlite est broyée, séchée puis calibrée dans une première étape. Le produit obtenu dit Perlite Ore est de couleur grise et de taille de l'ordre de 100 µm.

La Perlite Ore est ensuite expansée (1000°C/2 secondes) pour donner des particules plus ou moins blanches. Lorsque la température atteint 850-900 °C, l'eau emprisonnée dans la structure du matériau se vaporise et entraîne l'expansion du matériau par rapport à son volume d'origine. Les particules de perite expansées conformes à l'invention peuvent être obtenues par le procédé d'expansion décrit dans le brevet US 5,002,698.

De préférence, les particules de perlite utilisées seront broyées ; elles sont dans ce cas dites Expanded Milled Perlite (EMP). Elles ont de préférence une taille de particule définie par un diamètre médian D₅₀ allant de 0,5 à 50 µm et de préférence de 0,5 à 40 µm.

De préférence, les particules de perlite utilisées présentent une densité apparente non tassée à 25°C allant de 10 et 400 kg/m³ (Norme DIN 53468) et de préférence de 10 et 300 kg/m³.

De préférence, les particules de perlite expansée selon l'invention ont une capacité d'absorption d'eau mesurée au WET POINT allant de 200 à 1500%, de préférence 250 à 800%

Le Wet Point correspond à la quantité d'eau qu'il faut additionner à 1 g de particule pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :
WET POINT : masse exprimée en grammes pour 100g de produit correspondant à l'obtention d'une pâte homogène lors de l'addition d'un solvant à une poudre.
FLOW POINT : masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant :

### Protocole de mesure de l'absorption d'eau.

### 1) Matériel utilisé

Plaque de verre (25 x 25 mm)
Spatule (manche en bois et partie métallique (15 x 2,7mm)
Pinceau à poils de soie
Balance

### 2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule

On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

La quantité de particules de minéral amorphe expansé utilisée selon l'invention représente de 1 à 40 % et en particulier de 1 à 25% en poids du poids total de la composition.

### COMPOSES VOLATILS

Les compositions selon l'invention peuvent contenir en plus au moins un composé volatil.

Les composés volatils de l'invention sont des composés cosmétiques volatils, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Parmi les composés volatils utilisables conformément à l'invention, on peut citer les alcools volatils choisis de préférence parmi monoalcools inférieurs en C₁-C₅ peuvent être choisis parmi le méthanol, l'éthanol, propanol, l'isopropanol, le n-butanol, l'isobutanol le t-butanol et plus particulièrement l'éthanol.

A titre d'exemple de composés hydrocarbonés volatils utilisables dans l'invention, on peut également citer les huiles hydrocarbonées volatiles choisies parmi les huiles hydrocarbonées ayant de 8 à 16 atomes de carbones, et notamment les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars ou de Permetyls, les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées ; les alcanes linéaires volatils comme ceux décrits dans la demande de brevet de la société Cognis dans les demandes de brevet DE10 2008 012 457 et WO2008/155059.

A titre d'exemple de composés siliconés volatils utilisables dans l'invention, on peut citer les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane.

On peut également citer les huiles linéaires alkyltrisiloxanes volatiles de formule générale (I) : 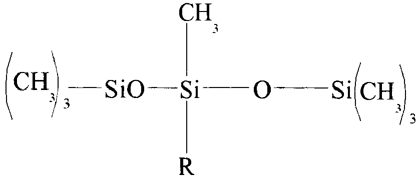 où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

### ACTIFS DEODORANTS / SELS OU COMPLEXES ANTITRANSPIRANTS

Selon une forme particulièrement préférée de l'invention, les compositions parfumantes contiennent en plus au moins un actif déodorant et/ou au moins un sel ou complexe anti-transpirant.

### a/ DEODORANTS

Au sens de la présente invention, on entend par "actif déodorant" toute substance capable de masquer, absorber, améliorer ou réduire l'odeur désagréable résultant de la décomposition de la sueur humaine par des bactéries.

Les actifs déodorants peuvent être des agents bactériostatiques ou des agents bactéricides comme le 2,4,4'-trichloro-2'-hydroxydiphényléther (^{®}Triclosan), le 2,4-dichloro-2'-hydroxydiphényléther, le 3',4',5'-trichlorosalicylanilide, la 1-(3',4'-dichlorophenyl)-3-(4'-chlorophenyl)urée (^{®}Tridocarban) ou le 3,7,11-triméthyldodéca-2,5,10-triénol (^{®}Farnesol) ; les sels d'ammonium quaternaires comme les sels de cetyltrimethylammonium, les sels de cétylpyridinium , le DPTA (acide 1,3-diaminopropanetétraacétique), le 1,2 decanediol (SIMCLARIOL de la société Symrise),

Parmi les actifs déodorants conformes à l'invention, on peut aussi citer également
- les sels de zinc comme le salicylate de zinc, le gluconate de zinc, le pidolate de zinc ; le sulfate de zinc, le chlorure de zinc, le lactate de zinc, le phénolsulfonate de zinc ;
- la chlorhexidine et ses sels;
- le bicarbonate de sodium ;
- l'acide salicylique et ses dérivés tels que l'acide n-octanoyl-5-salicylique
- les dérivés de glycérine comme par exemple le Caprylic/Capric Glycerides (CAPMUL MCM de Abitec), le Caprylate ou caprate de Glycerol (DERMOSOFT GMCY et DERMOSOFT GMC respectivement de STRAETMANS), le Polyglyceryl-2 Caprate (DERMOSOFT DGMC de STRAETMANS)
- les dérivés de biguanide comme les sels de polyhexaméthylène biguanide.
- Zéolites d'argent ou sans argent

En cas d'incompatibilité ou pour les stabiliser, certains des actifs mentionnés ci-dessus peuvent être incorporés dans des sphérules, notamment des vésicules ioniques ou non-ioniques et/ou des nanoparticules (nanocapsules et/ou nanosphères).

Les actifs déodorants peuvent être présents de préférence dans les compositions selon l'invention dans des concentrations pondérales allant 0,01 à 5% en poids par rapport au poids total de la composition.

Afin d'améliorer l'efficacité anti-transpirante de la composition, on peut utiliser en plus un ou plusieurs polymères anioniques hydrosolubles comprenant un acide Bronsted en particulier ceux dérivant de l'acide maléique et/ou de l'anhydride maléique qui sont décrits dans la demande de brevet WO02/49590.

### b/ SELS OU COMPLEXES ANTI-TRANSPIRANTS

Par "sel ou complexe anti-transpirant", on entend tout sel ou complexe qui, à lui seul, a pour effet de diminuer ou limiter le flux sudoral et/ou absorber la sueur humaine.

Les sels ou complexes anti-transpirants conformes à l'invention sont choisis généralement parmi les sels ou complexes d'aluminium et/ou de zirconium. Il sont de préférence choisis parmi les halohydrates de d'aluminium ; les halohydrates d'aluminium et de zirconium, les complexes d'hydroxychlorure de zirconium et d'hydroxychlorure d'aluminium avec ou sans un acide aminé tels que ceux décrits dans le brevet US-3792068.

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Les sels ou complexes anti-transpirants peuvent être présents dans la composition selon l'invention à raison d'environ 0,5 à 25% en poids par rapport au poids total de la composition.

### FORMES GALENIQUES

L'invention se rapporte également à une composition parfumante aqueuse comprenant dans un milieu cosmétiquement acceptable :
a) au moins 0,3% en poids par rapport au poids total de la composition d'une substance parfumante ;
b) au moins les dites particules ;
c) de l'eau

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

L'invention s'applique non seulement aux produits parfumants mais aussi aux produits de soin, de traitement de la peau, y compris du cuir chevelu, et des lèvres, contenant une substance odorante. La composition selon l'invention peut ainsi constituer une composition de parfumage, de soin, de traitement des matières kératiniques, et notamment se présenter sous forme d'eau fraîche, d'eau de toilette, d'eau de parfum, de lotion après-rasage, d'eau de soin, d'huile de soin siliconée ou hydrosiliconée, de crème anhydre. Elle peut également se présenter sous la forme d'une lotion biphasique parfumée (phase eau de toilette/phase huile hydrocarbonée et/ou huile siliconée).

L'invention porte également sur des compositions conditionnées dans un dispositif muni d'une paroi ajourée notamment une grille ; conditionnées dans un dispositif muni d'un applicateur à billes ("roll-on), conditionnées sous forme de bâtonnets (sticks) ; caractérisées par le fait qu'elles contiennent au moins des particules de perlite telle que définies précédemment. Elles contiennent à cet égard les ingrédients généralement utilisés dans ce type de produits et bien connus de l'homme de l'art.

Selon une autre forme particulière de l'invention, les compositions parfumantes selon l'invention peuvent être anhydres.

On entend par composition anhydre une composition contenant moins de 2 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Selon une forme particulière de l'invention, les compositions parfumantes selon l'invention peuvent également se présenter sous forme de crème anhydre.

Selon une forme particulière de l'invention, les compositions parfumantes selon l'invention peuvent également se présenter sous forme sous forme de poudre libre ou compactée.

Selon une forme particulière de l'invention, les compositions parfumantes selon l'invention peuvent également se présenter sous forme d'aérosol ou de spray dans un flacon pompe.

### EMULSIONNANTS

Les compositions selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112). Elles comportent en général un ou plusieurs émulsionnants.

### a) Emulsionnants huile-dans-eau

Comme émulsionnants pouvant être utilisés dans les émulsions huile-dans-eau ou émulsions triples huile-dans-eau-dans-huile, on peut citer par exemple les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

On peut citer également les mélanges émulsionnants alcool gras/alkylpolyglycoside tels que sont décrits dans les demandes WO92/06778, WO95/13863 et WO98/47610 comme les produits commerciaux vendus par la société SEPPIC sous les appellation MONTANOV ®.

### b) Emulsionnants eau-dans-huile

Parmi les émulsionnants pouvant être utilisés dans les émulsions eau-dans-huile ou émulsions triples eau-dans-huile-dans-eau-dans-huile ou émulsions triples, on peut citer à titre d'exemple les alkyl dimethicone copolyols répondant à la formule (I) suivante 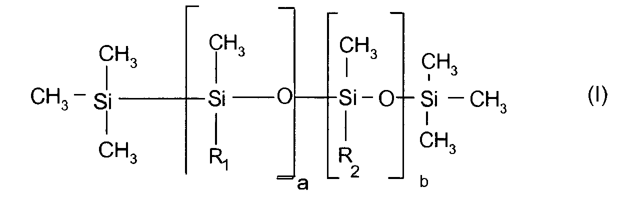 dans lesquelles :
R₁ désigne un groupement alkyle linéaire ou ramifié en C₁₂-C₂₀ et de préférence en C₁₂-C₁₈ ;
R₂ désigne le groupement :--CₙH₂ₙ--(-OC₂H₄-)ₓ--(-OC₃H₆-)_{y}--O-R₃,
R₃ désigne un atome d'hydrogène ou un radical akyle linéaire ou ramifié comportant de 1 à 12 atomes de carbone ;
a est un nombre entier allant de 1 à environ 500 ;
b désigne un nombre entier allant de 1 à environ 500 ;
n est un nombre entier allant de 2 à 12 et de préférence 2 à 5 ;
x désigne un nombre entier allant de 1 à environ 50 et de préférence de 1 à 30 ;
y désigne un nombre entier allant de 0 à environ 49 et de préférence 0 à 29 sous réserve que lorsque y est différent de zéro le ratio x/y est supérieur à 1 et de préférence varie de 2 à 11.

Parmi les émulsionnants alkyldimethicone copolyols de formule (I) préférés, on citera plus particulièrement le CETYL PEG/PPG-10/1 DIMETHICONE et plus particulièrement le mélange CETYL PEG/PPG-10/1 DIMETICONE AND DIMETHICONE (nom INCI) comme le produit vendu sous le nom commercial ABIL EM90 par la société GOLDSCHMIDT ou bien le mélange (POLYGLYCERYL-4-STEARATE and CETYL PEG/PPG-10 (AND) DIMETHICONE (AND) HEXYL LAURATE) comme le produit vendu sous le nom commercial ABIL WE09 par la même société.

Parmi les émulsionnants eau-dans-huile, on peut citer également les dimethicone copolyols répondant à la formule (II) suivante 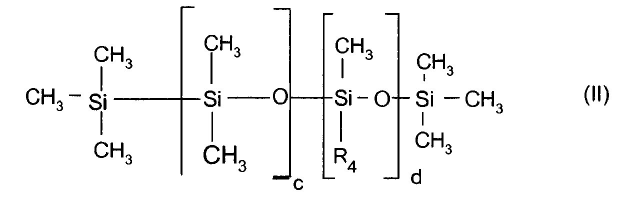 dans lesquelles
R₄ désigne le groupement :--CₘH₂ₘ--(-OC₂H₄-)ₛ--(-OC₃H₆-)ₜ--O-R₅,
R₅ désigne un atome d'hydrogène ou un radical akyle linéaire ou ramifié comportant de 1 à 12 atomes de carbone ;
c est un nombre entier allant de 1 à environ 500
d désigne un nombre entier allant de 1 à environ 500,
m est un nombre entier allant de 2 à 12 et de préférence 2 à 5 ,
s désigne un nombre entier allant de 1 à environ 50, et de préférence de 1 à 30 ;
t désigne un nombre entier allant de 0 à environ 50 et de préférence de 0 à 30 ; sous réserve que la somme s+t soit supérieure ou égal à 1.

Parmi ces émulsionnants dimethicone copolyols de formule (II) préférentiels on utilisera particulièrement le PEG-18/PPG-18 DIMETHICONE et plus particulièrement le mélange CYCLOPENTASILOXANE (and) PEG-18/PPG-18 DIMETHICONE (nom INCI) tel que le produit vendu par la société Dow Corning sous la dénomination commerciale Silicone DC 5225 C ou KF-6040 de la société Shin Etsu.

Selon une forme particulièrement préféré, on utilisera un mélange d'au moins un émulsionnant de formule (I) et d'au moins un émulsionnant de formule (II).

On utilisera plus particulièrement un mélange de PEG-18/PPG-18 Dimethicone et Cetyl PEG/PPG-10/1 DIMETHICONE et encore plus particulièrement un mélange de (CYCLOPENTASILOXANE (and) PEG-18/PPG-18 Dimethicone) et de Cetyl PEG/PPG-10/1 DIMETICONE and Dimethicone ou de (Polyglyceryl-4-stearate and Cetyl PEG/PPG-10 (and) Dimethicone (and) Hexyl Laurate).

Parmi les émulsionnants eau-dans-huile, on peut citer également les émulsionnants non ioniques dérivés d'acide gras et de polyol, les alkylpolyglycosides (APG), les esters de sucres et leurs mélanges.

Comme émulsionnants non ioniques dérivés d'acide gras et de polyol, on peut utiliser notamment les esters d'acide gras et de polyol, l'acide gras ayant notamment une chaîne alkyle en C₈-C₂₄, et les polyols étant par exemple le glycérol et le sorbitan.

Comme esters d'acide gras et de polyol, on peut citer notamment les esters d'acide isostéarique et de polyols, les esters d'acide stéarique et de polyols, et leurs mélanges, en particulier les esters d'acide isostéarique et de glycérol et/ou de sorbitan.

Comme esters d'acide stéarique et de polyols, on peut citer notamment les esters de polyéthylèneglycol comme le PEG-30 Dipolyhydroxystearate tel que le produit commercialisé sous le nom Arlacel P135 par la société ICI.

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, , le mélange d'isostéarate de sorbitan et d'isostéarate de polyglycérol (3 moles) commercialisé sous la dénomination Arlacel 1690 par la société Unigema. et leurs mélanges.

L'émulsionnant peut être choisi aussi parmi les alkylpolyglycosides ayant un HLB inférieur à 7, par exemple ceux représentés par la formule générale (1) suivante :

R-O-(G)x (1)

dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10 et de préférence de 1 à 4, et G désigne notamment le glucose, le fructose ou le galactose.

Le radical alkyle insaturé peut comprendre une ou plusieurs insaturations éthyléniques, et en particulier une ou deux insaturations éthyléniques.

Comme alkylpolyglycosides de ce type, on peut citer les alkylpolyglucosides (G=glucose dans la formule (I)), et notamment les composés de formule (I) dans laquelle R représente plus particulièrement un radical oléyle (radical insaturé en C18) ou isostéaryle (radical saturé en C18), G désigne le glucose, x est une valeur allant de 1 à 2, notamment l'isostéaryl-glucoside, l'oléyl-glucoside et leurs mélanges. Cet alkylpolyglucoside peut être utilisé en mélange avec un co-émulsionnant, plus spécialement avec un alcool gras et notamment un alcool gras ayant la même chaîne grasse que celle de l'alkylpolyglucoside, c'est-à-dire comportant de 14 à 24 atomes de carbone et ayant une chaîne ramifiée et/ou insaturée, et par exemple l'alcool isostéarylique quand l'alkylpolyglucoside est l'isostéaryl-glucoside, et l'alcool oléylique quand l'alkylpolyglucoside est l'oleyl-glucoside, éventuellement sous forme d'une composition autoémulsionnante, comme décrit par exemple dans le document WO-A-92/06778. On peut utiliser par exemple le mélange d'isostéaryl-glucoside et d'alcool isostéarylique, commercialisé sous la dénomination Montanov WO 18 par la société SEPPIC ainsi que le mélange octyldodécanol et octyldodecylxyloside commercialisé sous la dénomination FLUDANOV 20X par la société SEPPIC.

On peut également citer les polyoléfines à terminaison succinique, comme les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol ou le produit commercial CHEMCINNATE 2000.

La quantité totale en émulsionnants dans la composition sera de préférence dans la composition selon l'invention à des teneurs en matière active allant de 1 à 8% en poids et plus particulièrement de 2 à 6% en poids par rapport au poids total de la composition.

### PHASE AQUEUSE

La phase aqueuse des compositions parfumantes aqueuses de l'invention contient de l'eau et en général d'autres solvants solubles ou miscibles dans l'eau. Les solvants solubles ou miscibles dans l'eau comprennent les mono alcools à chaîne courte par exemple en C1-C4 comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine, le propane 1,3 diol.

### PHASE GRASSE

Les compositions selon l'invention peuvent contenir au moins une phase liquide organique non-miscible dans l'eau. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée comprend généralement au moins une huile volatile et/ou une huile non volatile et éventuellement au moins un agent structurant.

Par « huile », on entend un corps gras liquide à température ambiante (25 °C) et pression atmosphérique (760mm de Hg soit 105 Pa). L'huile peut être volatile ou non volatile.

Par « huile volatile », on entend au sens de l'invention une huile susceptible de s'évaporer au contact de la peau ou de la fibre kératinique en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par « huile non volatile », on entend une huile restant sur la peau ou la fibre kératinique à température ambiante et pression atmosphérique au moins plusieurs heures et ayant notamment une pression de vapeur inférieure à 10⁻³ mm de Hg (0,13 Pa).

L'huile peut être choisie parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges.

Plus précisément, par « huile hydrocarbonée », on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000 mPa.s, de préférence de 50 à 50 000 mPa.s et de préférence encore de 100 à 300 000 mPa.s.

A titre d'exemple d'huile volatile utilisable dans l'invention, on peut citer celles qui ont été citées précédemment dans le paragraphe se rapportant aux composants volatils.

A titre d'exemple d'huile non volatile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles les huiles de germe de blé, d'olive, l'huile d'amande douce, de palme, de colza, de coton, de luzerne, de pavot, de potimarron, de courge, de cassis, d'onagre, de millet, d'orge, de quinoa, de seigle, de carthame, de bancoulier, de passiflore, de rosier muscat, de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, le squalane ;
- les éthers de synthèse ayant de 10 à 40 atomes de carbone ;
- les esters de synthèse notamment d'acides gras comme les huiles de formule R₁COOR₂ dans laquelle R₁ représente le reste d'un acide gras supérieur linéaire ou ramifié comportant de 1 à 40 atomes de carbone et R₂ représente une chaîne hydrocarbonée notamment ramifiée contenant de 1 à 40 atomes de carbone avec R₁ + R₂ ≥ 10 comme par exemple l'huile de Purcellin (octanoate de cétostéaryle), l'isononanoate d'isononyle, le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les octanoates, décanoates ou ricinoléates d'alcools ou de polyalcools comme le dioctanoate de propylène glycol ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras liquides à température ambiante à chaîne carbonée ramifiée et/ou insaturée ayant de 12 à 26 atomes de carbone comme l'octyl dodécanol, l'alcool isostéarylique, le 2-butyloctanol, le 2-hexyl décanol, le 2-undécyl pentadécanol, l'alcool oléique ;
- les acides gras supérieurs tels que l'acide oléique, l'acide linoléique, l'acide linolénique ;
- les carbonates ;
- les acétates ;
- les citrates ;
- les huiles fluorées éventuellement partiellement hydrocarbonées et/ou siliconées comme les huiles fluorosiliconées, les polyéthers fluorés, les silicones fluorées telles que décrit dans le document EP-A-847752;
- les huiles siliconées comme les polydiméthylsiloxanes (PDMS) non volatiles, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates, et
- leurs mélanges.

### AGENT STRUCTURANT

Les compositions selon l'invention comprenant une phase grasse peuvent contenir en plus au moins un agent structurant de ladite phase grasse qui peut être choisi de préférence parmi les cires, les composés pâteux, les gélifiants lipophiles minéraux ou organiques et leurs mélanges.

Il est entendu que la quantité en ces composés peut être ajustée par l'homme du métier de manière à ne pas porter préjudice à l'effet recherché dans le cadre de la présente invention.

### CIRE(S)

La cire est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

### Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, Cire de Tournesol raffinée commercialisée sous la dénomination SUNFLOWER WAX par KOSTER KEUNEN, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone (C₃₀-₄₅ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Comme cire, on peut utiliser un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Comme micro-cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS, les produits commerciaux PERFOMALEN 400 POLYETHYLENE et PERFORMALENE 500-L POLYETHYLENE de NEW PHASE TECHNOLOGIES, le PERFORMALENE 655 POLYETHYLENE ou les cires de paraffine comme la cire ayant pour nom INCI , MICROCRISTALLINE WAX and SYNTHETIC WAX et vendue sous le nom commercial MICROLEASE par la Société SOCHIBO. ; les micro cires de polytétrafluoroéthylène telles que celles commercialisées sous les dénominations de Microslip 519® et 519 L® par la société MICRO POWDERS.

La composition selon l'invention comprendra de préférence une teneur en cire(s) allant de 3 % à 20% en poids par rapport au poids total de la composition, en particulier de 5 à 15%, plus particulièrement de 6 à 15%.

### Composés pâteux

Par « composé pâteux » au sens de la présente invention, on entend un composé gras lipophile à changement d'état solide/liquide réversible, présentant à l'état solide une organisation cristalline anisotrope, et comportant à la température de 23 °C une fraction liquide et une fraction solide.

Le composé pâteux est de préférence choisi parmi les composés synthétiques et les composés d'origine végétale. Un composé pâteux peut être obtenu par synthèse à partir de produits de départ d'origine végétale.

Le composé pâteux peut avantageusement choisi parmi :
- la lanoline et ses dérivés,
- les composés siliconés polymères ou non,
- les composés fluorés polymères ou non,
- les polymères vinyliques, notamment :
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyles ayant de préférence un groupement alkyle en C₈-C₃₀,
- les oligomères homo et copolymères d'esters vinyliques ayant des groupements alkyles en C₈-C₃₀, et
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- leurs mélanges.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en C4-C50 et un diol ou un polyol en C2-C50,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ®, et Risocast DA-L ®,
- les esters de dimère diol et dimère diacide, le cas échéant, estérifiés sur leur(s) fonction(s) alcool(s) ou acide(s) libre(s) par des radicaux acides ou alcools tels que le Plandool-G,
- leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

### GELIFIANTS LIPOPHILES

### Gélifiants minéraux

Comme gélifiant lipophile minéral, on peut citer les argiles éventuellement modifiées comme les hectorites modifiées par un chlorure d'ammonium en C10 à C22, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société DEGUSSA, CAB-O-SIL TS-530® par la société CABOT, des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

### Gélifiants organiques

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C1 à C6, et en particulier en C1 à C3 et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Comme gélifiant lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence; en particulier les résines de polyamides (notamment comprenant des groupes alkyles ayant de 12 à 22 atomes de carbone) telles que celles décrites dans US-A-5783657 dont le contenu est incorporé à titre de référence.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

### AGENTS DE SUSPENSION

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

### Poudre organique

Selon une forme particulière de l'invention, les compositions anti-transpirantes selon l'invention contiendront en plus une poudre organique.

On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les microsphères de poly methacrylate de methyle creuses (granulometrie : 6,5 - 10,5 p) commercialisées sous la dénomination GANZPEARL GMP 0800 par Ganz Chemical; micro-billes de copolymere methacrylate de methyle/dimethacrylate d'ethylene glycol (taille: 6.5-10.5 p) commercialisées sous la dénomination GANZPEARL GMP 0820 par Ganz Chemical ou MICROSPONGE 5640 par la société Amcol Health & Beauty Solutions; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

### AUTRES ADDITIFS

La composition de l'invention peut comprendre, en outre, tout additif usuellement utilisé dans le domaine des compositions parfumantes et/ou déodorantes choisi notamment parmi les actifs cosmétiques ou dermatologiques , les émollients ou adoucissants, les agents hydratants comme la glycérine, les agents apaisants comme l'α-bisabolol, l'allantoïne, aloes vera ; les vitamines, les propulseurs, des charges, les nacres, des paillettes, les colorants solubles dans le support de la composition, les stabilisants de couleur du parfum et leurs mélanges. Quand ils sont présents dans la composition de l'invention, ces additifs peuvent être présents en une quantité allant de 0,001 à 10 % et mieux de 0,01 à 5% en poids par rapport au poids total de la composition.

La composition de l'invention peut comprendre, en outre, des colorants solubles dans le support de ladite composition

Comme colorants solubles conformes à l'invention, on peut citer les colorants hydrosolubles ou hydrophiles tels que : le caramel, Yellow 5 , Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (CI 15850, CI 75470), Ext. Violet 2, Red 6-7, Ferric Ferrocyanide, Ultramarines, Acide Yellow 3 / Yellow 10, Acid Blue 3, Yellow 10.

Le ou les colorant (s) soluble (s) conformes à l'invention sont de préférence présents dans des quantités allant de 10-5 à 1 % du poids total de la composition, de préférence de 10-4 à 0,1% du poids total de la composition.

Comme stabilisants de la couleur de parfums, on citera le Tris(tétraméthylhydroxypipéridinol) citrate tel que produit vendu sous le nom « Tinoguard Q » par la société CIBA-GEIGY, le Sodium Benzotriazolyl Butylphenol Sulfonate comme le produit vendu sous le nom « TINOGUARD HS » par la société CIBA-GEIGY ; le Benzotriazolyl dodécyl p-Cresol comme le produit vendu sous le nom « TINOGUARD TL » par la société CIBA-GEIGY. comme le produit vendu sous le nom commercial « CIBAFAST H Liquid » par la société CIBA-GEIGY. Bumetrizole comme le produit vendu sous le nom « TINOGUARD AS » par la société CIBA-GEIGY.

Selon une forme particulière de l'invention, on utilisera en plus au moins un antioxydant et/ou au moins un agent peptisant de manière à améliorer la limpidité de la composition et/ou diminuer voire supprimer les phénomènes de précipitation à froid pouvant êtres causés par certains parfums et/ou améliorer la stabilité de la composition au stockage.

Parmi les antioxydants, on peut citer par exemple le BHA (tert-butyl-4-hydroxyanisole), le BHT (2,6-di-tert-butyl-p-crésol), les tocophérols comme la vitamine E et ses dérivés tels que l'acétate de tocophéryle. Ils sont utilisés à des concentrations allant de 0.01 % à 1 % par rapport au poids total de la composition.

Parmi les peptisants utilisables selon l'invention, on utilisera plus particulièrement l'huile de ricin hydrogénée oxyéthylénée.à 60 moles d'oxyde d'ethylène : Nom INCI : PEG-60 HYDROGENATED CASTOR OIL comme le produit vendu sous le nom commercial CREMAPHOR RH60 par la société BASF. Ils sont utilisés à des concentrations allant de 0,1% à 2 fois la concentration en concentré de parfum par rapport au poids total de la composition

Bien entendu l'homme du métier veillera à choisir les éventuels additifs complémentaires et/ou leur quantité de telle manière que les propriétés avantageuses de la composition selon l'invention ne soient pas ou substantiellement pas altérées par l'adjonction envisagée.

### AEROSOLS

Les compositions selon l'invention peuvent encore être pressurisées et être conditionnées dans un dispositif aérosol constitué par :
(A) un récipient comprenant une composition anti-transpirante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

Les compositions contenant les particules de perlite telles que définies précédemment et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations parfumantes suivantes; les quantités sont indiquées en pourcentages en poids :

### EXEMPLES

### TEST COMPARATIF DE REMANENCE DU PARFUM

La rémanence du parfum à 8 heures et 24 heures des aérosols déodorants suivantes a été évaluée :

| **Ingrédients** | **1 (invention)** | **2 (hors invention)** |
|---|---|---|
| Parfum (MASC E_0621398/01 commercialisé par Mane) | 1,0 | 1,0 |
| EXPANSED MILLED PERLITE (OPTIMAT 1430 OR - Word Minerals) | 1,0 | - |
| Ethanol | qsp | qsp 100 |
| Isobutane | 55 | 55 |

Les formules 1 et 2 sont pulvérisées 2 secondes dans des gobelets en carton. Ces gobelets sont stockées à température ambiante pendant 8 heures.

20 sujets évaluent alors l'intensité du parfum perçue, et répondent à la question suivante : l'intensité de l'odeur est selon vous : Nulle - Très faible - Faible - Moyen - Assez Fort - Fort - Très Fort"

La même question est de nouveau posée après 24 heures.

Un parfum est jugé rémanent s'il cumule au moins 60% des réponses parmi les items suivants : " Moyen - Assez Fort - Fort - Très Fort"

**Résultats à 8 heures**

| exemples | Nulle | Très faible | Faible | Moyen | Assez fort | Fort | Très fort | Ensemble des critères |
|---|---|---|---|---|---|---|---|---|
| 1 invention | 5% | 20% | 10% | 20% | 15% | 25% | 5% | 65% |
| 2 hors invention | 0% | 25% | 25% | 5,0% | 30% | 15% | 0% | 50% |

**Résultats à 24 heures**

| exemples | Nulle | Très faible | Faible | Moyen | Assez fort | Fort | Très fort | Ensemble des critères |
|---|---|---|---|---|---|---|---|---|
| 1 invention | 5% | 30% | 35% | 20% | 5% | 5% | 0% | 60% |
| 2 hors invention | 20% | 40% | 30% | 10% | 10% | 0% | 0% | 40% |

### EXEMPLE 3 : Stick anhydre déodorant parfumé

| Ingrédients (Nom INCI) | Quantités |
|---|---|
| CIRE DE POLYETHYLENE (PERFORMALENE 500-L POLYETHYLENE- New Phase technologie) | 4,1 |
| HOMOPOLYMERE DE L'ETHYLENE (PERFORMALENE 400 POLYETHYLENE -New Phase technologie) | 8,3 |
| PALMITATE D'ISOPROPYLE | 28,5 |
| ISO-HEXADECANE | 19,6 |
| DICAPRYLYL CARBONATE (CETIOL CC - COGNIS)) | 6,0 |
| MELANGE UNDECANE/TRIDECANE SELON L'EXEMPLE 1 OU L'EXEMPLE 2 DU WO2008/155059. | 10,0 |
| CYCLOPENTA DIMETHYLSILOXANE (DOW CORNING 245 FLUID- Dow Corning) | 0 |
| METHYL METHACRYLATE CROSSPOLYMER GANZPEARL GMP 0820 - Ganz Chemical) | 15,0 |
| EXPANSED MILLED PERLITE (OPTIMAT 1430 OR - Word Minerals) | 6,5 |
| PYRROLIDONE CARBOXYLATE DE ZINC MICRONISE (UCIB -Solabia) | 0,5 |
| PARFUM | 1,5 |

### EXEMPLE 4 : Roll-on anti-transpirant parfumé

| Ingrédients (Nom INCI) | Quantités |
|---|---|
| ALUMINUM CHLOROHYDRATE CHLORHYDROL 50 (SUMIT REHEIS) | 30 |
| EXPANSED MILLED PERLITE (OPTIMAT 1430 OR - Word Minerals) | 1 |
| STEARETH-100/PEG-136/HDI COPOLYMER (RHEOLATE FX 1100 - Elementis) | 1 |
| POLY DIMETHYLSILOXANE (VISCOSITE: 350 CST) (DOW CORNING 200 FLUID 350 CST - Dow Corning) | 0,5 |
| C14-22 ALCOHOLS (and) C12-20 ALKYL GLUCOSIDE MONTANOV L (Seppic) | 2,5 |
| PARFUM | 1,0 |
| Conservateur | qs |
| Eau permutée | qsp 100 |

### Exemple 5 : Aérosol déodorant parfumé

| Ingrédients (Nom INCI) | Quantités |
|---|---|
| TRIETHYL CITRATE CITROFLEX 2 (REILLY CHEMICALS | 1,0 |
| STEARALKONIUM BENTONITE TIXOGEL MP 250 (SUD CHEMIE RHEOLOG.) | 0,2 |
| ISOPROPYL PALMITATE | 0,9 |
| EXPANSED MILLED PERLITE (OPTIMAT 1430 OR - Word Minerals) | 2,6 |
| CYCLOPENTA DIMETHYLSILOXANE (DOW CORNING 245 FLUID- Dow Corning) | 9,0, |
| CYCLOPENTASILOXANE (and) DIMETHICONOL (DOW CORNING 1501 FLUID((DOW CORNING) | 1,3 |
| PARFUM | 1,0 |
| ISOBUTANE ( A-31 -AEROPRES) | qsp 100 |

### Exemple 6 : Crème anhydre déodorante parfumée

| Ingrédients (Nom INCI) | Quantités en % en poids |
|---|---|
| TRIETHYL CITRATE (CITROFLEX 2 -REILLY CHEMICALS) | 7,0 |
| PALMITATE D'ISOPROPYLE | 6,0 |
| EXPANSED MILLED PERLITE (OPTIMAT 1430 OR - Word Minerals) | 17,5 |
| CYCLOPENTASILOXANE (and) DIMETHICONOL DOW CORNING 1501 FLUID((DOW CORNING) | 9,0 |
| PARFUM | 1,0 |
| CYCLOPENTA DIMETHYLSILOXANE (DOW CORNING 245 FLUID- Dow Corning) | qsp 100 |

On disperse l'Optimat® 1430 OR dans le mélange des autres matières premières à la pâle. On obtient une pâte homogène.

## Revendications

1. Procédé cosmétique de parfumage d'une matière kératinique humaine consistant à appliquer sur ladite matière une composition parfumante comprenant dans un milieu cosmétiquement acceptable au moins 0,3% en poids d'au moins une substance parfumante et au moins des particules d'un matériau minéral expansé amorphe qui sont des particules de perlite **ayant une taille de particule définie par un diamètre médian D₅₀ allant de 0,5 à 50 µm.**

2. Procédé selon la revendication 1, où le matériau minéral amorphe expansé est issu d'au moins une roche volcanique.

3. Procédé selon la revendication **1 ou 2**, où le matériau minéral amorphe expansé issu d'au moins une roche volcanique est obtenu par expansion thermique d'une roche volcanique comprenant de 1 à 10% en poids d'eau et de préférence 1 à 5% en poids d'eau et moins de 10% en poids de roche cristalline par rapport au poids total de la composition de la roche, et de suivie d'un broyage.

4. Procédé **selon l'une quelconque des revendications 1 à 3**, où les particules de perlite sont broyées et ont une taille de particule définie par un diamètre médian D₅₀ allant de 0,5 à 40 µm.

5. Procédé selon l'une quelconque des revendications 1 à 4, où la quantité de particules de minéral amorphe expansé utilisée représente de **1 à 40 % et en particulier de 1 à 25% en poids du poids total de la composition**.

6. Procédé selon l'une quelconque des revendications 1 à 5, où la composition comprend en plus au moins un actif déodorant et/ou au moins un actif anti-transpirant.

7. Procédé selon l'une quelconque des revendications 1 à 6, où la composition parfumante est aqueuse.

8. Procédé selon l'une quelconque des revendications 1 à 7, où la composition se présente sous forme d'eau fraîche, d'eau de toilette, d'eau de parfum, de lotion après-rasage, d'eau de soin, d'huile de soin siliconée ou hydrosiliconée, de lotion bi-phasique, de brème anhydre.

9. Procédé selon l'une quelconque des revendications 1 à 8, où la composition est conditionnée :
(i) pressurisée dans un dispositif aérosol ou dans un flacon pompe ;
(ii) dans un dispositif muni d'un applicateur à bille ;
(iii) dans un dispositif muni d'une paroi ajourée notamment une grille ;
(iv) sous forme d'un bâtonnet (stick) ;
(v) sous forme de poudre libre ou compactée.

10. Composition parfumante aqueuse comprenant dans un milieu cosmétiquement acceptable :
a) au moins 0,3% en poids par rapport au poids total de la composition d'au moins une substance parfumante ;
b) au moins les particules minérales expansée telles que définies dans l'une quelconque des revendications 1 à 9,
c) de l'eau.

11. Composition s**elon la revendication 10**, comprenant dans un milieu cosmétiquement acceptable :
a) au moins 0,3% en poids par rapport au poids total de la composition d'au moins une substance parfumante ;
b) au moins les particules minérales expansée telles que définies dans l'une quelconque des revendications 1 à 9, **caractérisée par le fait qu'**elle se présente sous forme d'eau fraîche, d'eau de toilette, d'eau de parfum, de lotion après-rasage, d'eau de soin, d'huile de soin siliconée ou hydrosilioonée, de lotion bi-phasique, de crème anhydre.

12. Composition **selon la revendication 10 ou 11**, comprenant dans un milieu cosmétiquement acceptable :
a) au moins 0,3% en poids par rapport au poids total de la composition d'au moins une substance parfumante ;
b) au moins les particules minérales expansée telles que définies dans l'une quelconque des revendications 1 à 9, **caractérisée par le fait qu'**elle est conditionnée
(i) dans un dispositif muni d'un applicateur à bille ;
(ii) dans un dispositif muni d'une paroi ajourée notamment une grille ;
(iii) sous forme d'un bâtonnet (stick)

13. Procédé cosmétique de traitement des odeurs corporelles humaines en particulier axillaires, consistant à appliquer sur la surface de la peau une quantité efficace d'une composition parfumante telle que définie dans les revendications précédentes.

## Patentansprüche

1. Kosmetisches Verfahren zum Parfümieren eines humanen Keratinmaterials, bei dem man auf das Material eine Parfümierungszusammensetzung, die in einem kosmetisch unbedenklichen Medium mindestens 0,3 Gew.-% mindestens einer Parfümierungssubstanz und mindestens Teilchen eines amorphen expandierten Mineralmaterials, bei denen es sich um Perlitteilchen mit einer durch einen mittleren Teilchendurchmesser D₅₀ definierten Teilchengröße im Bereich von 0,5 bis 50 µm handelt, umfasst, aufbringt.

2. Verfahren nach Anspruch 1, wobei das expandierte amorphe Mineralmaterial von mindestens einem vulkanischen Gestein abstammt.

3. Verfahren nach Anspruch 1 oder 2, wobei das von mindestens einem vulkanischen Gestein abstammende expandierte amorphe Mineralmaterial durch thermisches Expandieren eines vulkanischen Gesteins, das 1 bis 10 Gew.-% Wasser und vorzugsweise 1 bis 5 Gew.-% Wasser und mindestens 10 Gew.-% kristallines Gestein, bezogen auf das Gesamtgewicht der Gesteinszusammensetzung, umfasst, und anschließendes Mahlen erhalten wird.

4. Verfahren nach einem der Ansprüche 1 bis 3, wobei die Perlitteilchen gemahlen sind und eine durch einen mittleren Teilchendurchmesser D₅₀ definierte Teilchengröße im Bereich von 0,5 bis 40 µm aufweisen.

5. Verfahren nach einem der Ansprüche 1 bis 4, wobei die verwendete Menge an expandierten amorphen Mineralteilchen 1 bis 40 Gew.-% und insbesondere 1 bis 25 Ges.-% des Gesamtgewichts der Zusammmensetzung ausmacht.

6. Verfahren nach einem der Ansprüche 1 bis 5, wobei die Zusammensetzung außerdem mindestens einen desodorierenden Wirkstoff und/oder mindestens einen schweißhemmenden Wirkstoff umfasst.

7. Verfahren nach einem der Ansprüche 1 bis 6, wobei die Parfümierungszusammensetzung wässrig ist.

8. Verfahren nach einem der Ansprüche 1 bis 7, wobei die Zusammensetzung in Form eines Eau fraiche, eines Eau de Toilette, eines Eau de Parfum, einer Aftershave-Lotion, eines Pflegewassers, eines gegebenenfalls Wasser enthaltenden Silikonpflegeöls, einer zweiphasigen Lotion oder einer wasserfreien Creme vorliegt.

9. Verfahren nach einem der Ansprüche 1 bis 8, wobei die Zusammensetzung:
(i) druckbeaufschlagt in einer Aerosolvorrichtung oder einer Pumpflasche;
(ii) in einer Vorrichtung mit einem Kugelapplikator;
(iii) in einer Vorrichtung mit einer durchbrochenen Wand, insbesondere einem Gitter;
(iv) in Form eines Stifts;
(v) in Form eines losen oder kompaktierten Puders abgepackt wird.

10. Wässrige Parfümierungszusammensetzung, umfassend in einem kosmetisch unbedenklichen Medium:
a) mindestens 0,3 Gew.-%, bezogen auf das Gesamtgewicht der Zusammensetzung, mindestens einer Parfümierungssubstanz;
b) mindestens die expandierten Mineralteilchen gemäß einem der Ansprüche 1 bis 9,
c) Wasser.

11. Zusammensetzung nach Anspruch 10, umfassend in einem kosmetisch unbedenklichen Medium:
a) mindestens 0,3 Gew.-%, bezogen auf das Gesamtgewicht der Zusammensetzung, mindestens einer Parfümierungssubstanz;
b) mindestens die expandierten Mineralteilchen gemäß einem der Ansprüche 1 bis 9,
**dadurch gekennzeichnet, dass** sie in Form eines Eau fraiche, eines Eau de Toilette, eines Eau de Parfum, einer Aftershave-Lotion, eines Pflegewassers, eines gegebenenfalls Wasser enthaltenden Silikonpflegeöls, einer zweiphasigen Lotion oder einer wasserfreien Creme vorliegt.

12. Zusammensetzung nach Anspruch 10 oder 11, umfassend in einem kosmetisch unbedenklichen Medium:
a) mindestens 0,3 Gew.-%, bezogen auf das Gesamtgewicht der Zusammensetzung, mindestens einer Parfümierungssubstanz;
b) mindestens die expandierten Mineralteilchen gemäß einem der Ansprüche 1 bis 9,
**dadurch gekennzeichnet, dass** sie
(i) in einer Vorrichtung mit einem Kugelapplikator;
(ii) in einer Vorrichtung mit einer durchbrochenen Wand, insbesondere einem Gitter;
(iii) in Form eines Stifts
abgepackt ist.

13. Kosmetisches Verfahren zur Behandlung von menschlichen Körpergerüchen, insbesondere Achselgerüchen, bei dem man auf die Oberfläche der Haut eine wirksame Menge einer Parfümierungszusammensetzung gemäß den vorhergehenden Ansprüchen aufbringt.

## Claims

1. Cosmetic method for scenting a human keratinous substance which consists in applying, to the said substance, a scenting composition comprising, in a cosmetically acceptable medium, at least 0.3% by weight of at least one scenting substance and at least particles of an expanded amorphous mineral material which are perlite particles having a particle size defined by a median diameter D₅₀ ranging from 0.5 to 50 µm.

2. Method according to Claim 1, where the expanded amorphous mineral material results from at least one volcanic rock.

3. Method according to Claim 1 or 2, where the expanded amorphous mineral material resulting from at least one volcanic rock is obtained by thermal expansion of a volcanic rock comprising from 1 to 10% by weight of water and preferably from 1 to 5% by weight of water and less than 10% by weight of crystalline rock, with respect to the total weight of the composition of the rock, followed by a milling.

4. Method according to any one of Claims 1 to 3, where the perlite particles are milled and have a particle size defined by a median diameter D₅₀ ranging from 0.5 to 40 µm.

5. Method according to any one of Claims 1 to 4, where the amount of particles of expanded amorphous mineral used represents from 1 to 40% by weight and in particular from 1 to 25% by weight of the total weight of the composition.

6. Method according to any one of Claims 1 to 5, where the composition additionally comprises at least one deodorant active principle and/or at least one antiperspirant active principle.

7. Method according to any one of Claims 1 to 6, where the scenting composition is aqueous.

8. Method according to any one of Claims 1 to 7, where the composition is provided in the form of an eau fraiche, eau de toilette, eau de parfum, aftershave lotion, care water, silicone or aqueous/silicone care oil, two-phase lotion or anhydrous cream.

9. Method according to any one of Claims 1 to 8, where the composition is packaged:
(i) pressurized in an aerosol device or in a pump-action spray;
(ii) in a device equipped with a ball applicator;
(iii) in a device equipped with an openwork wall, in particular a grating;
(iv) in the form of a stick;
(v) in the form of a loose or compact powder.

10. Aqueous scenting composition comprising, in a cosmetically acceptable medium:
a) at least 0.3% by weight, with respect to the total weight of the composition, of at least one scenting substance;
b) at least the expanded mineral particles as defined in any one of Claims 1 to 9,
c) water.

11. Composition according to Claim 10, comprising, in a cosmetically acceptable medium:
a) at least 0.3% by weight, with respect to the total weight of the composition, of at least one scenting substance;
b) at least the expanded mineral particles as defined in any one of Claims 1 to 9, **characterized in that** it is provided in the form of an eau fraîche, eau de toilette, eau de parfum, aftershave lotion, care water, silicone or aqueous/silicone care oil, two-phase lotion or anhydrous cream.

12. Composition according to claim 10 or 11, comprising, in a cosmetically acceptable medium:
a) at least 0.3% by weight, with respect to the total weight of the composition, of at least one scenting substance;
b) at least the expanded mineral particles as defined in any one of Claims 1 to 9, **characterized in that** it is packaged:
(i) in a device equipped with a ball applicator;
(ii) in a device equipped with an openwork wall, in particular a grating;
(iii) in the form of a stick.

13. Cosmetic method for treating human body odours, in particular axillary odours, which consists in applying, to the surface of the skin, an effective amount of a scenting composition as defined in the preceding claims.
